# EUROPEAN PATENT APPLICATION

(11) **EP 4 516 280 A1**
(43) Date of publication of application: **05.03.2025**
(21) Application number: 23020410.9
(22) Date of filing: 31.08.2023
(51) Int. Cl.: A61K 8/365, A61K 8/64, A61K 8/9717, A61K 8/9728, A61K 8/9789, A61K 36/82, A61Q 19/02, A61Q 19/08

(54) **COSMETIC COMPOSITIONS**

(71) Applicant: The Boots Company plc, Nottingham NG90 1BS (GB)
(72) Inventor: JOHNSON, Mark, Nottingham, NG15 8DQ (GB); O´CONNOR, Clare, West Hallam, Derbyshire DE7 6HG (GB); ELMS, Beverley Jane, Nottingham, Leicestershire, NG12 0FH (GB)
(74) Representative: Mukhtar, Amira

(57) **Abstract**

A composition comprising feruloyl oligopeptide-33 and a second tyrosinase inhibiting compound, and cosmetic methods of using said composition.

## Description

### Field of the Invention

The present invention relates to tyrosinase inhibiting compositions and methods of using such compositions. More particularly, the present invention relates to tyrosinase inhibiting compositions for topical application to improve the appearance of the skin.

### Background of the Invention

As skin ages, it undergoes changes which affect its appearance. For example, the skin can lose its tone and develop differently pigmented areas.

The development of unsightly darkened areas on the skin occurs for a number of reasons. For example, as the skin ages, there is an increasing tendency to develop age spots or senile lentigines. These are commonly seen as flat brown spots on the backs of the hands or temples. It is thought that these spots are associated with areas of chronic sun damage. These gradually develop over time. Other reasons for undesired pigmentation on the skin may include birthmarks, freckles, blemishes, discoloured scarring such as post inflammatory hyperpigmentation following a wound or skin blemish and hormonally induced conditions such as melasma.

Accordingly, it is desired to provide an improved skincare product for topical application which may be used to improve or enhance the appearance of the skin, particularly to reduce or inhibit the formation of skin pigmentation.

Current treatments for hyperpigmentation often encompass topical agents, chemical peels, and laser therapies. These approaches primarily target the overproduction of melanin, the pigment responsible for skin coloration.

Tyrosinase is a copper-containing enzyme present in plant and animal tissues that catalyses the production of melanin and other pigments from tyrosine by oxidation. Tyrosinase inhibiting compounds prevent or postpone the production of Tyrosinase, which in turn results in a reduction of the excessive production of melanin, resulting in lighter or more even skin pigmentation.

A number of ingredients, for example, hydroquinone, arbutin, one of the active components of bearberry (Arctostaphylos uva-ursi) extract, liquorice (Glycyrrhiza glabra) extract and it's active components, ascorbic acid and derivatives and kojic acid are amongst ingredients that have been shown or are reputed to have tyrosinase inhibiting activity. However, many of these skin whitening actives do not have a good safety profile or are not effective enough or have poor efficacy at levels safe for topical application to the skin.

Previous research has focussed on trying to find new compounds having improved efficacy and safety. However, there is a need to provide compositions with increased power with respect to reducing or inhibiting undesired skin pigmentation but which do not cause irritation to the skin.

### Summary of the Invention

The present inventors have found that a composition comprising feruloyl oligopeptide-33in combination with a second tyrosinase inhibiting compound provide an unexpected superior synergistic effect on the inhibition of tyrosinase. Furthermore, the compounds of the compositions are non-irritating and are well tolerated by the skin.

The present invention therefore provides in a first aspect a composition comprising:
(a) feruloyl oligopeptide-33; and
(b) a second tyrosinase inhibiting compound.

The combination of feruloyl oligopeptide-33 and a second tyrosinase inhibiting compound provides a synergistic tyrosinase inhibiting effect. The synergistic effect of the compounds in the composition of the first aspect allows significantly less total tyrosinase inhibiting compound to be employed in cosmetic compositions compared to standard quantities of tyrosinase inhibiting compound. The manufacturers recommended dosages of the materials are significantly higher than the levels used which have shown synergistic action. Accordingly, it is easier to formulate into a cosmetically acceptable composition for the consumer to provide a composition with pleasant skin feel which may be easily applied and spread evenly over the skin.

In a second aspect, the present invention provides a use of the composition of the first aspect for a topical cosmetic application to the skin.

In a third aspect, the present invention provides a use of the composition of the first aspect as a non-therapeutic cosmetic treatment to improve condition and/or appearance of the skin and/or imperfections (e.g. pigmentation).

In a fourth aspect, the present invention provides a method comprising applying the composition of the first aspect to the skin of a subject.

In a fifth aspect, the present invention also relates to a cosmetic method for improving the condition and/or appearance of the skin and/or imperfections (e.g. pigmentation), comprising topically administering the composition of the first aspect.

### Detailed Description of the Invention

The present invention is directed towards a composition having a synergistic combination of compounds for inhibiting tyrosinase.

Many of the most powerful tyrosinase inhibiting compounds do not have a good safety profile, and may cause dermatitis or even have sensitisation and irritant effects, whilst other safer alternatives are not as efficacious.

Previous research has focussed on trying to find new compounds having improved efficacy and safety, however the present inventors have surprisingly found that the combined action of certain compounds known to be safe for topical application to skin is greater than could have been predicted based on the performance of the compounds when used alone.

The composition of the present invention comprises (a) feruloyl oligopeptide-33.

The composition may comprise at least 0.0001 wt. % of feruloyl oligopeptide-33, such as for example at least 0.0001 wt. %, at least 0.00015 wt. %, at least 0.0002 wt. %, at least 0.00025 wt. %, or at least 0.0003 wt. %. The composition may comprise 1 wt. % of feruloyl oligopeptide-33 or less, such as for example 1 wt.% or less, 0.95 wt. % or less, 0.9 wt. % or less, 0.85 wt. % or less, 0.8 wt. % or less, 0.75 wt. % or less, 0.7 wt. % or less, 0.5 wt. % or less, 0.2 wt. % or less, 0.1 wt. % or less, or 0.01 wt. % or less. All percentages are of the composition of the present invention unless otherwise specified.

The feruloyl oligopeptide-33 may be provided in a solution comprising additional ingredients. Additional ingredients may comprise water, 1,2-hexanediol glycerin, glycine soja oil, disodium EDTA, hydrogenated phosphatidycholine, sodium oleate or combinations thereof.

The composition of the present invention comprises (b) a second tyrosinase inhibiting compound.

The second tyrosinase inhibiting compound may be selected from the group consisting of pelargonium extract, palmaria palmata extract, mulberry extract or green tea extract.

The second tyrosinase inhibiting compound may be pelargonium extract. The pelargonium extract may be pelargonium inquinans extract, pelargonium capitatum extract, pelargonium graveolans extract, pelargonium sidoides extract, or a combination thereof. The pelargonium extract may be pelargonium inquinans extract.

The composition may comprise at least 0.01 wt.% or more of the second tyrosinase inhibiting compound, such as for example at least at least 0.05 wt. %, at least 0.07 wt. %, 0.1 wt. %, at least 0.15 wt. %, at least 0.2 wt. %, at least 0.25 wt. %, or at least 0.3 wt. %. The composition may comprise 1 wt. % of the second compound or less, such as for example 1 wt.% or less, 0.95 wt. % or less, 0.9 wt. % or less, 0.85 wt. % or less, 0.8 wt. % or less, 0.75 wt. % or less, or 0.7 wt. % or less.

The combination of (a) feruloyl oligopeptide-33 and (b) a second tyrosinase inhibiting compound provides a synergistic tyrosinase inhibition effect. The synergistic tyrosinase inhibition effect is where the combination of (a) and (b) provides a greater tyrosinase inhibition effect than the sum of the individual tyrosinase inhibition effects of (a) and (b) when adjusted for the concentration of (a) and (b).

If feruloyl oligopeptide-33 at a concentration of 100 wt.% has a tyrosinase inhibition of 90 % and a second tyrosinase inhibiting compound has a tyrosinase inhibition of 60 %, then the sum of the individual tyrosinase inhibition effects of the first and second compound when adjusted for the concentration in a blend comprising 50% of the first compound and 50 % of the second compound would be 0.5 × 90 + 0.5 × 60 for a total of 75 %. This is also referred to as the predicted tyrosinase inhibition. For the above example, the present invention requires that the combination of the first compound and the second compound has a tyrosinase inhibition of greater than 75 % i.e., greater than the predicted tyrosinase inhibition.

The second tyrosinase inhibiting compound may be pelargonium extract. Where the second tyrosinase inhibiting compound is pelargonium extract, the composition may comprise (c) a third tyrosinase inhibiting compound. The third tyrosinase inhibiting compound may be selected from palmaria palmata extract, hydrolysed yeast extract, mandelic acid, sophora angustifolia extract, mulberry extract, or green tea extract.

The third compound may be present in an amount of at least 0.01 wt.%, such as for example at least 0.05 wt. %, at least 0.07 wt. %, at least 0.1 wt. %, at least 0.15 wt. %, at least 0.2 wt. %, at least 0.25 wt. %, or at least 0.3 wt. %. The third compound may be present in an amount of at least 1 wt.% or less, such as 0.95 wt. % or less, 0.9 wt. % or less, 0.85 wt. % or less, 0.8 wt. % or less, 0.75 wt. % or less, 0.7 wt. % or less, 0.65 wt. % or less, 0.6 wt. % or less, 0.55 wt. % or less, or 0.5 wt. % or less.

The combination of (a), (b), and (c) may provide a further synergistic tyrosinase inhibition effect.

The weight ratio of (a):(b) may be from 1:10 to 10:1, such as from 1:5 to 5:1 or from 1:3 to 3:1. The weight ratio of (a):(c) may be from 1:10 to 10:1, such as from 1:5 to 5:1 or from 1:3 to 3:1. The weight ratio of (a):(b):(c) may be from 1:10:10 to 10:1:1, such as from 1:5:5 to 5:1:1 or from 1:3:3 to 3:1:1.

The composition may further comprise skincare actives known to have benefit on the skin. For example, the composition may further comprise actives e.g. salicylic acid, vitamins e.g. Vitamin A or retinol, anti-wrinkle or anti-ageing actives such as peptides and promoters of collagen and/or elastin production, hydroxy acids, anti-oxidants, antiinflammatories, anti-microbials e.g. triclosan, other skin-lightening actives such as mulberry or ascorbic acid derivatives, anti-fungals, skin conditioners e.g. panthenol, organic or inorganic sunscreens e.g. butyl methoxy dibenxoyl methane and titanium dioxide, plant extracts and the active principals from these, skin identical lipids such as ceramides, moisturisers and other materials to promote the repair of the skin barrier such as hyaluronic acid and other suitable materials that are known to those skilled in the art.

The composition of the present invention may be a cosmetic composition. A cosmetic composition is a product designed for use by a consumer and is preferably a skincare cosmetic composition, more preferably a facial skincare cosmetic composition.

The composition of the present invention may be incorporated may include lipsticks, foundations, lip balms, face creams, eye creams, hand creams, face washes, body washes, shower gels, bath foams, mousses, foaming cleansers, toner cleansers, aftersuns, moisturisers, sun protection and face masks. Suitable composition types comprise aqueous or oily solutions or dispersions or emulsions or gels, including creams, serums, pastes, lotions, milks, ointments, salves, sticks, spray, roll-on, powders, aqueous gels, suspension dispersions, emulsions and impregnated adhesive patches.

The composition of the present invention may be a single-phase system or multiple phase system. The composition may include but is not limited to liquids, gels, balms, oils or solids. Single or multiple phase compositions are envisaged. Multiple phase systems include but are not limited to microemulsions, emulsions, and products with discrete separate phases. Emulsions include water-in-oil emulsions, oil-in-water emulsions and multiple emulsions (water-in-oil-in-water emulsions or oil-in-water-in-oil emulsions for example). Products with discrete separate phases include bi or triphasic systems where the individual water or oil phases can be visibly seen.

The composition of the invention may comprise a cosmetically acceptable carrier. The cosmetically acceptable carrier may be water-based, oil-or wax-based, or emulsion-based.

Where the carrier is emulsion-based, the composition may be in the form of a water-in-oil, an oil-in-water, a water-in-oil-in-water or an oil-in-water-in-oil emulsion.

Where the carrier is water-based, water may be present at a level of about 40% or more, about 45% or more, about 50% or more, about 55% or more, or about 60% or more by weight of the composition.

Where the carrier is oil-or wax-based, the oil and/or wax may be present at a level of about 15% or more, about 20% or more, about 30% or more, about25% or more, about 35% or more, or about 40% or more by weight of the composition.

The carrier may be water based and may comprise de-ionized water, purified water, natural spring water, rose water or the like. Mixtures of more than one of these may also be used. De-ionized or purified water may be used.

Water-based carriers may be 100% water or may comprise components other than water. These may be components known for use in cosmetic compositions. They may include, but are not limited to, agents such as water-soluble moisturising agents, conditioning agents, anti-microbials, humectants (e.g. glycerin) and/or other water- soluble skin care actives.

The carrier may be oil or wax based. The oil may be natural oil or synthetic oil, but preferably is natural oil such as a vegetable oil or a nut oil. The oil may be liquid or solid. The wax is preferably a natural wax.

Combinations of one or more oils and/or one or more waxes may be used.

Where the composition is an emulsion, it comprises an oil phase and a water phase. The oil phase of an emulsion can be provided by any suitable oily component. Suitable oils for the oil phase may comprise for example: (a) hydrocarbon oils, such as paraffin or mineral oils; (b) waxes, such as beeswax or paraffin wax; (c) natural oils, such as sunflower oil, apricot kernel oil, shea butter or jojoba oil; (d) silicone oils, such as dimethicone, silicone elastomer, cyclomethicone or cetyl dimethicone; (e) fatty acid esters and ethers, such as isopropyl palmitate or isopropyl myristate and polypropylene glycol-15 stearyl ether; (f) fatty alcohols, such as cetyl alcohol or stearyl alcohol; or (g) combinations thereof, for example, the blend of waxes available commercially under the trade name Cutina^{®} (BASF).

The emulsion may comprise 0.1 % to 55% by weight of the emulsion of oil phase. The emulsion may comprise 3% to 25% by weight of the emulsion of oil phase, such as from 5% to 20% by weight of the emulsion of oil phase. The emulsion may comprise 10% to 50% by weight of the emulsion of oil phase, such as from 25-50% by weight of the emulsion of oil phase.

The oil phase of the emulsion may comprise oil, wax, butter or combinations thereof. Waxes and butters are hydrocarbons that consist of long aliphatic alkyl chains and may include aromatic groups. They are generally lipophilic and typically solid or malleable at room temperature. Melting points vary depending on the alkyl chain, chain length and associations. Silicone waxes are the preferred type of suitable waxes based on alkylmethylsiloxane. Where present, wax or butter may be present at up to 40% of the oil phase of the emulsion. The oil phase may contain wax or butter at levels of up to 20% of the oil phase of the emulsion. The oil phase may contain wax or butter at levels of up to 10% of the oil phase of the emulsion.

Oils may comprise liquid oils. Liquid oils may comprise avocado oil, Camellia oil, turtle bean oil, macadamia nut oil, corn oil, mink oil, olive oil, Canoga oil, egg yolk oil, sesame seed oil, Persic oil, wheat germ oil, Camellia sasanqua oil, castor oil, linseed oil, safflower oil, sunflower oil, grapeseed oil, apricot oil, shea oil, sweet almond oil, cotton oil, evening primrose oil, palm oil, perilla oil, hazelnut oil, soybean oil, peanut oil, tea seed oil, kaya oil, rice bran oil, rapeseed oil, alfalfa oil, Chinese tung tree wood oil, Japanese tung tree wood oil, jojoba oil, germ oil, poppyseed oil, pumpkin oil, blackcurrant oil, millet oil, barley oil, quinoa oil, rye oil, candlenut oil, passionflower oil, musk rose oil, triglycerine, glyceryl trioctanoate, glyceryl triisopalmitate and combinations thereof.

Oils may comprise solid oils/fats. Solid oils/fats may comprise cocoa butter, coconut butter, horse fat, hardened coconut oil, palm oil, beef tallow, mutton tallow, hardened beef tallow, palm kernel oil, lard, Japan wax kernel oil, hardened oil, Japan wax, shea butter, and hardened castor oil.

Waxes may comprise beeswax, candelilla wax, carnauba wax, lanolin, lanolin acetate, liquid lanolin, sugar cane wax, fatty acid isopropyl lanolin, hexyl laurate, reduced lanolin, jojoba wax, hard lanolin, polyoxyethylene (hereinafter referred to as POE), lanolin alcohol ether, POE lanolin alcohol acetate, lanolin fatty acid polyethylene glycol, and POE hydrogenated lanolin alcohol ether.

Oils may comprise ester oils. Ester oils may be selected from the group consisting of C12-C15 alcohols benzoate, tridecyl salicylate, dibutyl adipate, isopropyl myristate, cetyl octoate, octyldodecyl myristate, isopropyl palmitate, butyl stearate, hexyl laurate, myristyl myristate, decyloleate, hexyldecyl dimethyl octoate, cetyl lactate, myristyl lactate, lanolin acetate, isocetyl stearate, isocetyl iso-stearate, 12-hydroxy cholesteryl stearate, di-2-ethylhexylic acid ethyleneglycol, dipentaerythritol fatty acid ester, N-alkylglycol monoisostearate, neopentylglycol dicaprate, diisostearyl malate, glyceryl di-2-heptyl undecanate, tri-methylol propane tri-2-ethylhexyl acid, tri-methylol propane triisostearate, pentaerythritol tetra-2-ethylhexyl acid, glyceryl tri-2-ethyl-hexanoate, tri-methylol propane triisostearate, cetyl-2-ethylexanoate, 2-ethylhexyl-palmitate, glycerine trimyristate, glyceride tri-2-heptyl undecatoic acid, methyl ester of castor oil fatty acid, oleate oil, acetoglyceride, palmitate-2-heptyl undecyl, diisopropyl adipate, N-lauroyl-L-glutamic acid-2-octyldodecil ester, di-2-heptylundecyl adipate, di-2-ethylhexyl sebacate, myristate-2-hexyldecyl, palmitate-2-hexyldecyl, adipate-2-hexyldecyl, diisopropyl sebacate, succinate-2-ethylhexyl and combinations thereof.

Fatty acids may comprise lauric acid, myristic acid, palmitic acid, stearic acid, behenic acid, oleic acid, 12-hydroxy-stearic acid, undecylenic acid, lanolin fatty acid, isostearic acid, linoleic acid, linolenic acid, and eicosapentaenoic acid.

Fatty alcohols may comprise lauryl alcohol, cetyl alcohol, stearyl alcohol, behenyl alcohol, myristyl alcohol, oleyl alcohol, cetostearyl alcohol, monostearyl glycerine ether (batyl alcohol), 2-decyltetradecinol, lanolin alcohol, cholesterol, phytosterol, hexyldodecanol, isostearyl alcohol, octyldodecanol.

Where the composition is an emulsion, it may comprise an emulsifier. The composition may comprise from 0.1 % to 10% emulsifier, for example from 0.25% to 7.5% or from 0.5% to 5%, emulsifier by weight of the composition. The emulsifier helps disperse and suspend the aqueous water phase within the oil phase.

The composition of the present invention may comprise one or more emulsifiers. Suitable emulsifiers include all those suitable for the purpose and known by those skilled in the art for use in skin care products. Where the emulsion is a water-in-oil emulsion, these emulsifiers have an HLB value of or less than 14, more preferably from 2 to 14, and still more preferably from 4 to 14. Where the emulsion is an oil-in-water emulsion, the surfactants may have a HLB value of greater than 8, such as from 8 to 18, or from 10 to 16.

The one or more emulsifier may comprise various non-ionic and anionic emulsifying agents such as sugar esters and polyesters, alkoxylated sugar esters and polyesters, C₁-C₃₀ fatty acid esters of C₁-C₃₀ fatty alcohols, alkoxylated derivatives of C₁-C₃₀ fatty acid esters of C₁-C₃₀ fatty alcools, alkoxylated ethers of C₁-C₃₀ fatty alcohols, polyglyceryl esters of C₁-C₃₀ fatty acids, C₁-C₃₀ esters of polyols, C₁-C₃₀ ethers of polyols, alkyl phosphates, polyoxyalkylene fatty ether phosphates, fatty acid amides, acyl lactylates, soaps, and combinations thereof. The one or more emulsifier may comprise non-silicon-comprising emulsifiers such as polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, steareth-20, ceteareth-20, PPG-2 methyl glucose ether distearate, ceteth-10, diethanolamine cetyl phosphate, glyceryl stearate, PEG-100 stearate, and combinations thereof.

The composition of the invention may further comprise one or more surfactants, including but not limited to, anionic surfactants (e.g. sodium lauryl sulphate, sodium laureth sulphate, ammonium laureth sulphate, disodium laureth sulfosuccinate and sodium C12-15 pareth-12 carboxylate), amphoteric/zwitterionic surfactants (e.g. cocamidopropyl betaine, sodium cocoamphoacetate and cocamidopropyl hydroxysultaine), non-ionic surfactants (e.g. cocamide DEA, cocamide MEA, decyl glucoside, lauryl glucoside), and cationic surfactants (e.g. cetrimonium chloride, behentrimonium chloride and benzalkonium chloride). The composition of the invention may not, in some examples, comprise sulphates as surfactants. Where one or more surfactants are present in the composition, the one or more surfactants may be present in an amount of about 0.1% to about 10% by weight of the composition, about 0.25% to about 7.5% by weight of the composition, or about 0.5% to about 5% by weight of the composition. Where one or more surfactants are present in the composition, the one or more surfactants are present in an amount of about 0.5% to about 5% by weight of the composition.

The composition of the invention may further comprise one or more preservatives. The one or more preservative may be selected from the group consisting of 2-bromo-2nitropropane-1,3-diol (bronopol, commercially available under the trade name Myacide RTM), benzyl alcohol, benzoic acid, sodium benzoate, diazolidinyl urea, imidazolidinyl urea, methyl paraben, phenoxyethanol, ethyl paraben, propyl paraben, sodium methyl paraben, sodium dehydroacetate, dehydroacetic acid, polyhexamethylenebiguanide hydrochloride, isothiazolone, chlorhexidine digluconate, chlorphenesin, sodium propyl paraben and combinations thereof. In some examples, the composition of the invention may not comprise parabens. In examples where one or more preservatives are present in the composition, the one or more preservatives may be present in an amount of about 0.001% to about 10% by weight of the composition, about 0.01% to about 8% by weight of the composition, or about 0.1% to about 5% by weight of the composition. In examples where one or more preservatives are present in the composition, the one or more preservatives are present in an amount of about 0.05% to about 8% by weight of the composition.

The composition of the invention may further comprise one or more chelating agents or sequestering agents, including but not limited to, ethylenediamine tetraacetic acid (EDTA) and salts thereof (e.g. dipotassium EDTA, disodium EDTA or tetrasodium EDTA), sodium phytate, trisodium ethylene diamine disuccinate and/or tetrasodium glutamate diacetate. Where one or more chelating agents are present in the composition, the one or more chelating agents may be present in an amount of about 0.001% to about 10% by weight of the composition, about 0.01% to about 8% by weight of the composition, or about 0.1% to about 5% by weight of the composition. Where one or more chelating agents are present in the composition, the one or more chelating agents are present in an amount of about 0.05% to about 8% by weight of the composition.

The composition of the invention may further comprise one or more humectants, including but not limited to glycerin, propylene glycol, propanediol, butylene glycol, pentylene glycol, hexylene glycol, hexanediol, dipropylene glycol, polyethylene glycol, sorbitol, sodium hyaluronate, urea, xylitol, lactitol, fructose, glucose, mannose, xylose, honey, pyrrolidone, and carboxylic acid and salts thereof. When present, the one or more humectants may be present in the composition in an amount of about 0.01% to about 20% by weight of the composition, about 0.1% to about 10%, or about 0.5% to about 7% by weight of the composition.

The composition of the invention may further comprise one or more emollients, including but not limited to PPG-15 stearyl ether, ethylhexyl stearate, cetyl dimethicone, octyldodecanol, PPG-20 methyl glucose ether, isopropyl myristate, isopropyl paltimate, isopropyl laurate, isodecyl laurate, isodecyl neopentanoate, isohexadecane, pentaerythrityl tetraisostearate, caprylic/capric triglyceride, canola oil, sunflower oil (*Helianthus annus*), olive oil (*Olea europea*), cottonseed oil (*Gossypium herbaceum*)*,* jojoba oil (*Simmondsia chinensis*), shea butter (*Butyrospermum parkii*), cocoa butter (*Theobroma cacao*), cupuacu butter (*Theobroma grandiflorum*), avocado oil (*Persea gratissima*), liquid paraffin, dimethicone, phenyl trimethicone, cyclopentasiloxane, dimethiconol and petrolatum. When present, the one or more emollients may be present in the composition in an amount of about 0.01% to about 20% by weight of the composition, about 0.1% to about 10%, or about 0.5% to about 7% by weight of the composition.

The composition of the invention may further comprise one or more vitamins. For example, the composition may further comprise vitamin B, vitamin B1 to vitamin B12, vitamin C, vitamin D, vitamin E, vitamin K, vitamin H, derivatives thereof, provitamins thereof (e.g. pro-vitamin B5 (panthenol)), or combinations thereof. Where one or more vitamins are present in the composition, the one or more vitamins may be present in a amount of about 0.0001% to about 50% by weight of the composition, about 0.001% to about 10% by weight of the composition, about 0.01% to about 8% by weight of the composition, or about 0.1% to about 5% by weight of the composition. Where one or more vitamins are present in the composition, the one or more vitamins are present in an amount of about 0.1% to about 5% by weight of the composition. Where one or more vitamins are present, the vitamin is vitamin C, vitamin E and/or a derivative thereof.

The composition may comprise one or more antioxidant agent. The antioxidant agent(s) may be a plant, algal or fungal extract, or derivative thereof, comprising one or more species which provide an antioxidant benefit, such as flavonoid species; phenolic acid species; stilbene species; lignin species, or combinations thereof. Alternatively, the antioxidant agent(s) may be synthetic or nature identical.

The one or more antioxidant agent may comprise one or more flavonoid species. Flavonoid species include flavones, flavonols, flavanones, flavanols, anthocyanidins, anthocyanins, proanthocyanidins, flavans, isoflavones and isoflavonoids. Some specific examples of flavonoid species are catechins (catechin, epicatechin, gallocatechin, epigallocatechin, epicatechin gallate, epigallocatechin gallate), quercetin, rutin, hesperidin and genistein.

The one or more antioxidant agent may comprise one or more carotenoid species. Carotenoid species include xanthophylls and carotenes. Specific examples of carotenoids are β-carotene, alpha-carotene, gamma carotene, beta-cryptoxanthin, lycopene, lutein, and zeaxanthin.

The one or more antioxidant agent may comprise one or more enzyme species. Antioxidant enzymes include glutathione peroxidases, catalases and superoxide dismutase (SOD).

The one or more antioxidant agent may comprise one or more glutathione species. Glutathione species include reduced glutathione (GSH, or L-glutathione), and oxidized glutathione (GSSG), the inactive state.

The one or more antioxidant agent may comprise one or more lipid associated chemical species. Lipid associated chemical species include Ubiquinol-10, N-acetyl cysteine, lipoic acid.

The one or more antioxidant agent may comprise one or more saponine or steroid species. Saponin species may be plant-derived, or isolated from marine organisms such as sea cucumber. Examples of saponin sources are Sapindaceae, including its defining genus Sapindus (soapberry or soapnut), Aesculus hippocastanum (horse chesnut), Aceraceae (maples), Hippocastanaceae, Gynostemma pentaphyllum (Cucurbitaceae), ginseng or red ginseng (Panax, Araliaceae) Manilkara zapota fruit (also known as sapodillas) and Nerium oleander (Apocynaceae). Saponins may be derived from the leaves, stems, roots, bulbs, blossom and fruits of these plants.

The one or more antioxidant agent may comprise one or more vitamins, such as vitamin C or vitamin E.

Plants provide a rich and cheap source of antioxidant agents and are therefore an efficient source of said agents. Naturally occurring antioxidant agents may therefore be used.

However, the same or similar actives can be also prepared synthetically. The term "antioxidant agent" is therefore intended to cover antioxidant agents including synthetic polyphenols, such as synthetic analogues of naturally occurring antioxidant agents. Thus, chemically synthesized or purified polyphenols and mixtures thereof may be used in place of plant extracts. Polyphenols may be synthesized or extracted from natural sources by any suitable method known to those skilled in the art, particularly using food-grade solvents. Liquid and solid (e.g. granulate or powder form) extracts are suitable.

Extracts (e.g. aqueous or alcoholic) may be obtained from plant parts including but not limited to leaves, raw or cooked whole fruit, berries and vegetables, nuts, the skins of fruit, fruit flesh, fruit rind, peel, pips, cones (e.g. hops), seeds or stones, bark, buds, flowers or parts thereof, including petals and pollen, roots, rhizomes and tubers, and stems.

Plant extracts may be selected from the group consisting of essential oils, extracts from leaves, extracts from stems, extracts from petals, extracts from seeds, extracts from roots, extracts from pollen, and combinations thereof. Extracts (e.g. lyophilised extracts) from leaves may be used. Extracts may be prepared by microwave extraction (e.g, *Quercus petraea* fruit extract).

The term "antioxidant agent" is intended to mean a plant extract or derivative thereof comprising flavonoid species, including flavones, flavonols, flavanones, flavanols, anthrocyanidins and isoflavonoids; phenoic acid species; stilbenes; lignans or combinations thereof, which provides an antioxidant benefit. Plants provide a rich and cheap source of antioxidant agents and are therefore an efficient source of said agents. Similar actives can be also prepared synthetically and as such are analogues. The term "antioxidant agent" is also intended to cover said analogues. The plant extract may be selected from the group consisting of essential oils, extracts from leaves, extracts from stems, extracts from petals, extracts from seeds, extracts from roots, extracts from pollen, and combinations thereof.

Antioxidant agent(s) may be selected from the group consisting of dimethylmethoxy chromanol (such as that sold under the trade name of LIPOCHROMAN^{®} Molecule from Lipotec), *Myrtus communis* leaf extract (such as that sold under the trade name of DEXOSINE BIO^{®} from Silab), green tea extract (e.g. *Camellia sinensis* leaf extract), *Quercus petraea* fruit extract (such as that sold under the trade name of Phytessence French Oak^{™} from Croda), *Camellia japonica* extract (e.g. a *Camellia japonica* flower extract such as that sold under the trade name of RedSnow^{®} from Clariant), emblica extract (e.g. *Phyllanthus emblica* fruit extract (also known as *Emblica officinalis* (Gooseberry) fruit extract)), ginkgo extract (e.g. *Ginkgo biloba* leaf extract), ferulic acid (also known as hydroxycinnamic acid), and combinations thereof.

Where one or more antioxidants are present in the composition, the one or more antioxidants are present in an amount of from about 0.1 % to about 5% by weight of the composition.

The composition of the invention may further comprise one or more of extracts of ginseng (e.g. *Panax ginseng*), raspberry, oregano (e.g. *Origanum vulgare*), white tea (e.g. *Camellia sinensis*), red tea, Mohani tea, black tea, Oolong tea, yellow tea, jasmine tea, Pu Erh tea, blueberry (e.g. *Vaccinium cyanococcus*), rosemary (e.g. *Rosmarinus officialis*)*,* grape, including grape seed (e.g. *Vitis vinifera*), fennel (e.g. *Foeniculi fructus*), *Caragana sinica,* majaoram (e.g. *Origanum majorana*)*,* crocus (e.g. *Crocus sativus*), apple (e.g. *Malus domestica*), coffee, green coffee, cherry (e.g. *Prunus avium*), snow algae (e.g. *Chlamydomonas nivalis*), moringa (e.g. *Moringa oleilera*)*,* ginger, magnolia (e.g. *Magnolioideae virginiana*)*,* French saffron, edelweiss (e.g. *Leontopodium alpinium*)*,* white lotus (e.g. nymphaea alba), turmeric root, marshmallow (e.g. *Althaea officianlis*)*,* burdock (e.g. *Arctium lappa*)*,* bilberry (e.g. *Vaccinium myrtillus*)*,* cranberry (e.g. *Vaccinium oxycoccus),* pomegranate (e.g. *Punica granatum*), sage (e.g. *Salvia officianlis*), thyme (e.g. *Thymus vulgaris*), sunflower (e.g. *Helianthus annus*)*,* wild carrot (e.g. *Daucus carota*), hop (e.g. *Humulus lupulus*), witch hazel (e.g. *Hamamelis*)*,* oak (e.g. *Quercus*), Camellia (e.g. *Theacea*)*,* red clover (e.g. *Tritolium pratense*), flax (e.g. *Linium usitatissiumum*)*,* lemon (e.g. *Citrus limon*)*,* birch (e.g. *Betula*)*,* cornflower (e.g. *Centaurea cyanus*), geranium, polygonum, soy (e.g. *Glycine max*)*,* or.

The composition of the invention may further comprise one or more sunscreen agents, including but not limited to inorganic sunscreen agents (e.g. microfine titanium dioxide, microfine zinc oxide, iron oxides, talcs and/or boron nitride) and organic sunscreen agents (e.g. p-aminobenzoic acids, esters and derivatives thereof (e.g. 2-ethylhexyl p-dimethyl-aminobenzoate), methoxycinnamate esters (e.g., 2-ethylhexyl p-methoxycinnamate, 2-ethoxyethyl p-methoxycinnamate or α,β-di-(p-methoxycinnamoyl)-α'-(2ethylhexanoyl)-glycerin), benzophenones (e.g. oxybenzone), dibenzoylmethanes (e.g. 4-(tert-butyl)-4'-methoxydibenzoylmethane), 2-phenylbenzimidazole-5 sulfonic acid and salts thereof, alkyl-β,β-diphenylacrylates (e.g. alkyl α-cyano-β,β-diphenylacrylates such as octocrylene) triazines (such as 2,4,6-trianilino-(p-carbo-2-ethyl-hexyl-1-oxy)-1,3,5 triazine), and/or camphor derivatives (such as methylbenzylidene camphor). Where one or more sunscreen agents are present in the composition, the one or more sunscreen agents may be present in an amount of about 0.01 to about 10% by weight of the composition.

The composition of the invention may further comprise one or more pH adjusting agents, including but not limited to potassium hydroxide, sodium hydroxide, aminomethyl propanol, sodium citrate and/or triethanolamine. The composition of the invention may have a pH from about 3 to about 10, e. g. from about 4 to about 8, or from about 5 to about 7. Where one or more pH adjusting agents are present in the composition, the one or more pH adjusting agents may be present in an amount of from about 0.01 to about 10% by weight of the composition.

The composition of the invention may further comprise one or more thickeners or gelling agents. For example, when the composition is in the form of a gel, the composition may comprise one or more thickeners or gelling agents. Examples of thickeners/gelling agents that can be used in the present invention include, but are not limited to, acrylic acid polymers (e.g. available commercially under the trade name Carbopol or Ultrez (Lubrizol), modified celluloses (e.g. hydroxyethylcellulose available commercially under the trade name Natrosol from Hercules) hydroxypropylmethyl cellulose, amine oxides, block polymers of ethylene oxide and propylene oxide (e.g. those available from BASF Wyandotte under the trade name "Pluronic"), PVM, MA, decadiene crosspolymer (e.g. available under the trade name Stabilez 60), ethoxylated fatty alcohols, salt (e.g. magnesium chloride, sodium chloride), Aristoflex AVC, phthalic acid amide, xanthan gum, sodium polyacrylate, polyvinyl alcohols, fatty alcohols, and/or alkyl galactmanans (e.g. available under the trade name N-Hance from Hercules. Where one or more thickeners/gelling agents are present in the composition, the one or more thickeners/gelling agents may be present in an amount of about 0.01 to about 10% by weight of the composition.

The compositions of the invention may further comprise one or more perfumes and/or colourings. The composition may further comprise one or more conditioning agents.

The composition may comprise waxes such as cocoa butter suitably in an amount of from 1 % to 99% by weight of the composition. The composition may comprise suitable, cosmetically acceptable diluents, carriers and/or propellants such as dimethyl ether. The composition may also comprise pearlising agents such as stearic monoethanolamide and/or mica, suitably in an amount of from 0.01% to 10% by weight of the composition.

The invention further provides a use of the composition of the present invention for a topical cosmetic application to the skin.

The present invention also provides a use of the composition of the present invention as a non-therapeutic cosmetic treatment to improve condition and/or appearance of the skin and/or imperfections (e.g. pigmentation).

The present invention also provides a method comprising applying the composition of the present invention to the skin of a subject.

The present invention also provides a cosmetic method for improving the condition and/or appearance of the skin and/or imperfections (e.g. pigmentation), comprising topically administering the composition of the present invention.

### Examples

### Tyrosinase Inhibition Screening

The tyrosinase inhibition test was used to determine the tyrosinase inhibition ability of the compounds. The test comprises adding the test compound to a solution of L-dihydroxyphenylalanine (L-(DOPA)) and mushroom tyrosinase. L-(DOPA) is converted to dopamine by the mushroom tyrosinase, causing a brown colour to form. Inhibition of this reaction prevents the brown colour formation. The lighter the colour at the end of the reaction, the stronger is the inhibitory effect of the active. Absorbance data may be collected using a plate reacted set up to have a kinetic read every 1 minute for 30 minutes, where absorbance is read at 470 nm.

### Compound Screening

12 known tyrosinase inhibiting compounds (A to J) with good safety profiles were tested to determine their individual tyrosinase inhibition activities, alongside a control (sphinganine, K) which does not produce a positive reaction.

| Concentration 0.1% | | Tyrosinase Inhibition (%) |
|---|---|---|
| A | Pelargonium extract | 60.58 |
| B | Mandelic acid | 80.279 |
| C | Green tea extract | 53.511 |
| D | Palmaria Palmata extract | 18.1 |
| E | Feruloyl oligopeptide-33 | 41.765 |
| F | Hydrolysed yeast extract/glycolipids (HYE) | 53.34 |
| G | Cannabidiol isolate (CBD) | 9.134 |
| H | Dipotassium glycyrrhizate (DPG) | 54.567 |
| I | Mulberry extract | 39.8 |
| J | Sophora angustifolia extract (sophora extract) | 46.85 |
| K | Sphinganine | -5.78 |

Various combinations of compounds A-M were then tested.

### Combination Screening

The results showed that there were several combinations that exhibited synergy when compared to the individual actives rather than an additive effect when compared to the results for the individual ingredients.

Predicted tyrosinase inhibition is the predicted additive effect from the values determined in the compound screening equal to the sum of the individual tyrosinase inhibition effects of (a) and (b) when adjusted for the concentration of (a) and (b).

Actual tyrosinase inhibition was experimentally determined as detailed above. 100% actual tyrosinase inhibition (TI) indicates that the tyrosinase was completely inhibited.

| **Comp No** | **Ingredient (%)** | | | **Predicted TI (%)** | **Actual TI (%)** | **Inc. (%)** |
|---|---|---|---|---|---|---|
| **1** | Feruloyl Oligopeptide-33 (0.28) | Pelargonium extract (0.072) | - | 55.0 | 100 | 45.0 |
| **2** | Feruloyl Oligopeptide-33 (0.70) | Green tea extract (0.30) | - | 45.0 | 100 | 55.0 |
| **3** | Feruloyl Oligopeptide-33 (0.70) | palmaria palmata extract (0.30) | - | 56.0 | 91.0 | 35.0 |
| **4** | Feruloyl Oligopeptide-33 (0.70) | Mulberry extract (0.30) | - | 58.4 | 72.1 | 13.7 |
| **5** | Feruloyl Oligopeptide-33 (0.30) | Mulberry extract (0.30) | - | 24.6 | 54.8 | 30.2 |
| **6** | Feruloyl Oligopeptide-33 (0.70) | Green tea extract (0.30) | - | 58.8 | 68.3 | 9.50 |
| **7** | Feruloyl Oligopeptide-33 (0.34) | Pelargonium extract (0.31) | Sophora extract (0.34) | 49.1 | 63.7 | 29.1 |
| **8** | Feruloyl Oligopeptide-33 (0.33) | Pelargonium extract (0.33) | DPG (0.33) | 52.3 | 62.1 | 9.87 |
| **9** | Feruloyl Oligopeptide-33 | Pelargonium extract (0.33) | Mulberry extract | 47.4 | 74.4 | 27.1 |
| | (0.33) | | (0.33) | | | |
| **10** | Feruloyl Oligopeptide-33 (0.33) | Pelargonium extract (0.33) | HYE (0.33) | 51.5 | 62.2 | 10.8 |
| **11** | Feruloyl Oligopeptide-33 (0.33) | Pelargonium extract (0.33) | Mandelic acid (0.33) | 60.7 | 70.0 | 9.25 |
| **12** | Feruloyl Oligopeptide-33 (0.33) | Pelargonium extract (0.33) | Palmaria Palmata extract (0.33) | 40.2 | 50.7 | 10.4 |

The combinations of compounds in compositions 1 to 12 demonstrate an enhanced tyrosinase inhibitory effect greater than that expected from the additive effects of the compounds in isolation. A synergistic effect is therefore demonstrated for compositions of the present invention.

### Example Composition 1 - Anti-ageing Hand Cream

| Material | % w/w |
|---|---|
| Aqua | To 100% |
| Ethylhexyl methoxycinnamate | 7.5 |
| C12-C15 alkyl benzoate | 6.0 |
| Glycerin | 5.8 |
| Butyl methoxydibenzoylmethane | 3.0 |
| Butyrospermum parkii | 2.0 |
| Ethylhexyl salicylate | 2.0 |
| C18-36 acid glycol ester | 1.5 |
| Glyceryl stearate | 1.0 |
| Cetyl alcohol | 1.0 |
| PEG-100 stearate | 0.98 |
| Cyclopentasiloxane | 1.0 |
| Dimethicone | 0.90 |
| Polyacrylamide emulsion | 2.0 |
| Cyclohexasiloxane | 0.50 |
| Tocopheryl acetate | 0.50 |
| Butylene glycol | 0.40 |
| Dimethiconol | 0.50 |
| Acrylates/vinyl isodecanoate crosspolymer | 0.12 |
| Palmitoyl pentapeptide-3 | 0.00015 |
| Pelargonium extract | 0.10 |
| Feruloyl Oligopeptide-33 | 0.50 |
| Lupinus albus | 0.021 |
| Tetrasodium EDTA | 0.050 |
| Preservative | q.s |
| Perfume | q.s |

### Example Composition 2 - Water in Oil Emulsion

| Material | % w/w |
|---|---|
| Dimethicone | 13.83 |
| Water | 33.71 |
| Glycerin | 5.00 |
| Dimethicone crosspolymer & Dimethcone | 32.31 |
| Butylene glycol | 2.60 |
| PEG/PPG-18/18 dimethicone & Polyglyceryl -4 isostearate & Hexyl laurate | 3.00 |
| Cetyl PEG/PPG-10/1 dimethicone | 2.00 |
| Magnesium sulphate | 0.60 |
| Phenoxyethanol & Caprylyl glycol & Ethylhexylglycerin | 0.55 |
| Pelargonium extract | 0.01 |
| Feruloyl Oligopeptide-33 | 0.01 |
| Mulberry extract | 0.01 |
| Alcohol denat. | 4.00 |

### Method of manufacture

1. In the main vessel add Dimethicone, Dimethicone crosspolymer, PEG/PPG-18/18 dimethicone & polyglyceryl-4 isostearate & hexyl laurate and Cetyl PEG/PPG-10/1 dimethicone to make the oil phase.
2. Separately weigh out water, magnesium sulphate, glycerin, phenoxyethanol & caprylyl glycol & ethylhexylglycerin, pelargonium extract. mulberry and feruloyl oligopeptide 33
3. Add the water phase to the oil phase slowly with constant stirring at high speed (creating a vortex). Continue stirring for 5 minutes.
4. Homogenise the product for 5 minutes at 3500 rpm using a Silverson mixer or equivalent.

### Example Composition 3 - Oil in water emulsion

| Material | % w/w |
|---|---|
| Dimethicone | 5.50 |
| Water | 76.55 |
| Glycerin | 5.00 |
| Dimethicone crosspolymer & Dimethicone | 1.00 |
| Phenoxyethanol & Caprylyl glycol & Ethylhexylglycerin | 0.80 |
| Glyceryl stearate & PEG-100 stearate | 2.00 |
| Cetearyl alcohol | 2.00 |
| Sodium polyacrylate | 0.60 |
| Xanthan gum | 0.10 |
| Tetrasodium EDTA | 0.05 |
| Feruloyl Oligopeptide-33 | 2.00 |
| Pelargonium extract | 0.20 |
| Resveratrol | 0.20 |
| Alcohol denat. | 4.00 |

### Method of manufacture

1. To water add glycerin and dissolve tetrasodium EDTA.
2. Using homogenisation sprinkle in xanthan gum and continue to homogenise for 5 minutes or until hydrated.
3. Heat water phase to 70-75°C.
4. In a separate vessel weigh out oil phase and heat to 70-75°C. (Dimethicone, cetearyl alcohol, glyceryl stearate & PEG-100 stearate) When at temperature stir in the sodium polyacrylate.
5. With both phases at 70-75°C add the oil phase to the water phase and homogenise for 2 minutes.
6. Add dimethicone crosspolymer & dimethicone and homogenise for 2 minutes.
7. Cool to room temperature.
8. Stir in Phenoxyethanol & Caprylyl glycol & Ethylhexylglycerin, Pelargonium extract and Feruloyl oligopeptide-33
9. Pre dissolve Resveratrol in Alcohol denat. and stir into the bulk.
10. Make to weight with water and stir smooth.

### Example Composition 4 - Oil in water emulsion containing sunscreens

| Material | % w/w |
|---|---|
| C12-15 alkyl benzoate | 5.00 |
| Butyl methoxydibenzoylmethane | 3.00 |
| Ethylhexyl methoxycinnamate | 5.00 |
| Cetearyl alcohol | 2.00 |
| Glycerin | 2.00 |
| Glyceryl stearate & PEG-100 stearate | 3.00 |
| Dimethicone | 1.50 |
| PEG-20 stearate | 0.50 |
| Phenoxyethanol & Caprylyl glycol & Ethylhexylglycerin | 0.40 |
| Carbomer | 0.20 |
| Potassium hydroxide | 0.06 |
| Tetrasodium EDTA | 0.05 |
| Water | 70.89 |
| Pelargonium extract | 0.5 |
| Feruloyl Oligopeptide-33 | 0.20 |
| Resveratrol | 0.20 |
| Alcohol denat. | 4.00 |

### Method of manufacture

1. To water add glycerine and dissolve tetrasodium EDTA.
2. Using homogenisation sprinkle in carbomer and continue to homogenise for 5 minutes or until hydrated.
3. Heat water phase to 70-75°C.
4. In a separate vessel weigh out oil phase and heat to 70-75°C. (Dimethicone, cetearyl alcohol, glyceryl stearate & PEG-100 stearate, C12-15 alkyl benzoate, Butyl methoxydibenzoylmethane, Ethylhexyl methoxycinnamate, PEG-20 stearate)
5. With both phases at 70-75°C add the oil phase to the water phase and homogenise for 2 minutes.
6. Add Potassium hydroxide and homogenise for 2 minutes.
7. Cool to room temperature.
8. Stir in Phenoxyethanol & Caprylyl glycol & Ethylhexylglycerin and feruloyl oligopeptide 33 and Pelargonium extract
9. Dissolve Pinus Pinaster Bark Extract in a small amount of water by warming to 40-50°C and stir into the bulk.
10. Pre dissolve Resveratrol in Alcohol denat. and stir into the bulk.
11. Make to weight with water and stir smooth.

### Example Composition 5 - Gel

| Material | % w/w |
|---|---|
| Glycerin | 5.00 |
| Propanediol | 2.00 |
| Acrylates/C10-30 alkyl acrylate crosspolymer | 1.00 |
| Phenoxyethanol & Caprylyl glycol & Ethylhexylglycerin | 0.40 |
| Potassium hydroxide | 0.29 |
| Tetrasodium EDTA | 0.05 |
| Water | 84.86 |
| Aqua & Glycerin & Argania Spinosa Leaf Extract | 2.00 |
| Pelargonium extract | 0.20 |
| Feruloyl Oligopeptide-33 | 0.20 |
| Alcohol denat. | 4.00 |

### Method of manufacture

1. To water add glycerine and propanediol and dissolve tetrasodium EDTA.
2. Using homogenisation sprinkle in acrylates/C10-30 alkyl acrylate crosspolymer and continue to homogenise for 5 minutes or until hydrated.
3. Stir in potassium hydroxide to form gel.
4. Stir in Phenoxyethanol & Caprylyl glycol & Ethylhexylglycerin and Aqua & Glycerin & Argania Spinosa Leaf Extract, pelargonium extract and Feruloyl oligopeptide 33
5. Make to weight with water and stir smooth.

## Claims

1. A composition comprising:
(a) feruloyl oligopeptide-33; and
(b) a second tyrosinase inhibiting compound.

2. The composition of claim 1, wherein the second tyrosinase inhibiting compound is selected from the group consisting of pelargonium extract, palmaria palmata extract, mulberry extract or green tea extract.

3. The composition of claim 1 or claim 2, wherein feruloyl oligopeptide-33 is present in an amount of at least 0.0001 wt. % of the composition.

4. The composition of any one of claims 1 to 3, wherein feruloyl oligopeptide-33 is present in an amount of from 0.0001 wt. % to 1 wt. % of the composition.

5. The composition of any one of claims 1 to 4, wherein the second compound is present in an amount of from at least 0.01 wt.% of the composition.

6. The composition of any one of claims 1 to 5, wherein the second compound is present in an amount of from 0.01 wt.% to 1 wt. % of the composition.

7. The composition of any one of claims 1 to 6, wherein the second compound is pelargonium extract and the composition further comprises (c) a third compound selected from palmaria palmata extract, hydrolysed yeast extract, mandelic acid, sophora angustifolia extract, mulberry extract, or green tea extract.

8. The composition of any one of claims 7, wherein the third compound is present in an amount of from at least 0.01 wt.% of the composition.

9. The composition of claim 7 or claim 8, wherein the third compound is present in an amount of from 0.01 wt.% to 1 wt. % of the composition.

10. A use of the composition of any one of claims 1 to 9 for a topical cosmetic application to the skin.

11. A use of the composition of any one of claims 1 to 9 as a non-therapeutic cosmetic treatment to improve condition and/or appearance of the skin and/or imperfections.

12. A method comprising applying the composition of any one of claims 1 to 9 to the skin of a subject.

13. A cosmetic method for improving the condition and/or appearance of the skin and/or imperfections, comprising topically administering the composition of any one of claims 1 to 9.
